# EUROPEAN PATENT APPLICATION

(11) **EP 3 111 878 A1**
(43) Date of publication of application: **04.01.2017**
(21) Application number: 15755720.8
(22) Date of filing: 26.02.2015
(51) Int. Cl.: A61B 90/00, A61B 1/00, A61B 1/04

(54) **SURGERY SYSTEM AND METHOD OF AVOIDING INTERFERENCE WITH MEDICAL INSTRUMENT**

(30) Priority: 27.02.2014 JP 2014036794
(71) Applicant: Olympus Corporation, Hachioji-shi Tokyo 192-8507 (JP)
(72) Inventor: NICHOGI, Masao, Hachioji-shi, Tokyo 192-8507 (JP); KIKUCHI, Satoru, Hachioji-shi, Tokyo 192-8507 (JP)
(74) Representative: Gunzelmann, Rainer
(86) International application number: PCT/JP2015/055724
(87) International publication number: WO 2015/129834

(57) **Abstract**

A surgical system (100) includes: a medical device (1); an organ measuring means (7) for measuring a three-dimensional position of an organ (D) in the interior of a body cavity; a non-interference-region setting portion (10) that sets, on the basis of the three-dimensional position of the organ (D), a non-interference region, which is a space in the interior of the body cavity excluding the organ (D); a medical-device detecting means (8) for detecting a three-dimensional position of the medical device (1) in the interior of the body cavity; a medical-device-occupying-region calculating portion (11) that calculates, on the basis of the three-dimensional position of the medical device (1), a medical-device occupying region of the medical device (1) in the interior of the body cavity; an interference predicting portion (12) that predicts interference between the medical device (1) and the organ (D) on the basis of the positional relationship between the non-interference region and the medical-device occupying region; and an alerting portion (13) that alerts an operator when interference is predicted by the interference predicting portion (12).

## Description

### {Technical Field}

The present invention relates to a surgical system and a medical-device-interference avoidance method.

### {Background Art}

In the related art, a method has been employed in laparoscopic surgery, in which an endoscope and a treatment tool such as forceps or the like are inserted into the abdominal cavity at the same time from separate holes formed in the body wall of a patient, and in which treatment is performed by using both the endoscope and the treatment tool. Because the endoscope and the treatment tool inserted into the abdominal cavity are manipulated independently of each other, interference sometimes occurs between the endoscope and the treatment tool, thus hindering the surgery. Therefore, endoscopes provided with a function for avoiding interference with the treatment tool have been proposed (for example, see Patent Literature 1). In Patent Literature 1, interference sensor that senses interference with a treatment tool or a peripheral tissue is provided in an inserted portion of an endoscope, and angles of joints provided in the inserted portion are controlled on the basis of sensing of the interference sensor so as to achieve a flexed shape that does not cause interference between the inserted portion and the treatment tool.

### {Citation List}

### {Patent Literature}

{PTL 1} Japanese Unexamined Patent Application, Publication No. 2004-81277

### {Summary of Invention}

### {Technical Problem}

However, the endoscope of Patent Literature 1 adjusts the joint angles after interference between the inserted portion and the treatment tool or the peripheral tissue is sensed, and thus, it is not possible to avoid interference between the inserted portion and the treatment tool or between the inserted portion and the peripheral tissue in advance. In addition, Patent Literature 1 includes no description of a means for avoiding interference in advance. Furthermore, although the endoscope of Patent Literature 1 employs a gyro sensor, a strain gauge, or a piezoelectric sensor as the interference sensor, it is technically difficult to stably sense interference with, in particular, a soft organ with high sensitivity by using such interference sensor, and thus, there is a problem in that such interference sensor is inadequate as a means for avoiding interference with the peripheral tissue.

The present invention has been conceived in light of the above-described circumstances, and an object thereof is to provide a surgical system and a medical-device-interference avoidance method with which it is possible to reliably prevent, in advance, the occurrence of interference between a medical device and peripheral tissues in the body cavity.

### {Solution to Problem}

In order to achieve the above-described object, the present invention provides the following solutions.

A first aspect of the present invention is a surgical system including: a medical device that can be inserted into an interior of a body cavity of a living body; an organ measuring means for measuring a three-dimensional position of an organ in the interior of the body cavity; a non-interference-region setting portion that sets, on the basis of the three-dimensional position of the organ measured by the organ measuring means, a non-interference region, which is a space in the interior of the body cavity excluding the organ; a medical-device detecting means for detecting a three-dimensional position of the medical device in the interior of the body cavity; a medical-device-occupying-region calculating portion that calculates, on the basis of the three-dimensional position of the medical device detected by the medical-device detecting means, a medical-device occupying region that is occupied by the medical device in the interior of the body cavity; an interference predicting portion that predicts interference between the medical device and the organ on the basis of the positional relationship between the non-interference region set by the non-interference-region setting portion and the medical-device occupying region calculated by the medical-device-occupying-region calculating portion; and an alerting portion that alerts an operator when interference between the medical device and the organ is predicted by the interference predicting portion.

With the first aspect of the present invention, the three-dimensional position of the medical device in the interior of the body cavity is detected by the medical-device detecting means, and the medical-device occupying region is calculated by the medical-device-occupying-region calculating portion on the basis of the detected three-dimensional position. At the same time, the three-dimensional position of the organ in the interior of the body cavity is measured by the organ measuring means, and the non-interference-region setting portion sets, on the basis of the measured three-dimensional position, the non-interference region, that is, the portion of the interior of the body cavity in which the organ does not exist.

Then, the alerting portion alerts the operator when interference between the medical device and the peripheral tissue positioned outside the non-interference region is predicted by the interference predicting portion on the basis of the positional relationship between the medical-device occupying region and the non-interference region. By doing so, the operator can take an avoidance action, such as more cautiously manipulating the medical device, moving the medical device away from a potentially interfering peripheral tissue, or the like, before the medical device interferes with the peripheral tissue, and thus, it is possible to reliably prevent, in advance, the occurrence of interference between a medical device and peripheral tissues in the body cavity.

A second aspect of the present invention is a surgical system including: a medical device that can be inserted into an interior of a body cavity of a living body; an organ measuring means for measuring a three-dimensional position of an organ in the interior of the body cavity; a non-interference-region setting portion that sets, on the basis of the three-dimensional position of the organ measured by the organ measuring means, a non-interference region, which is a space in the interior of the body cavity excluding the organ; a medical-device detecting means for detecting a three-dimensional position of the medical device in the interior of the body cavity; a medical-device-occupying-region calculating portion that calculates, on the basis of the three-dimensional position of the medical device detected by the medical-device detecting means, a medical-device occupying region that is occupied by the medical device in the interior of the body cavity; an interference predicting portion that predicts interference between the medical device and the organ on the basis of the positional relationship between the non-interference region set by the non-interference-region setting portion and the medical-device occupying region calculated by the medical-device-occupying-region calculating portion; and a medical-device control portion that applies a restriction on the operation of the medical device when interference between the medical device and the organ is predicted by the interference predicting portion.

With the second aspect of the present invention, the operation of the medical device is restricted by the medical-device control portion when interference between the medical device and the peripheral tissue is predicted by the interference predicting portion, and thus, it is possible to reliably prevent, in advance, the occurrence of interference between a medical device and peripheral tissues in the body cavity.

The above-described second aspect may additionally be provided with an alerting portion that alerts an operator when interference between the medical device and the organ is predicted by the interference predicting portion.

In the above-described first and second aspects, the alerting portion may output a notification signal that is noticeable by at least one of the vision, the auditory sense, and the sense of touch of the operator.

By doing so, it is possible to alert the operator by means of a simple method.

The above-described first and second aspects may be provided with a cylindrical trocar that is inserted into the interior of the body cavity via a hole formed in a body surface of the living body and into which the medical device can be inserted, wherein the organ measuring means may include: a stereo camera that is provided in a distal-end portion of the trocar and that acquires a pair of viewpoint images by capturing a viewing field in front of the distal end of the trocar from two viewpoints that are different from each other; a viewpoint-position detecting portion that detects a position and an orientation of the stereo camera in the interior of the body cavity; and a computation portion that acquires, by means of calculation, a three-dimensional position of the organ in the interior of the body cavity on the basis of the pair of viewpoint images acquired by the stereo camera and the position and the orientation of the stereo camera detected by the viewpoint-position detecting portion.

By doing so, the pair of viewpoint images acquired by the stereo camera contain the information about the distances from the distal-end portion of the trocar to the individual positions of the peripheral tissue surface. The computation portion can acquire the three-dimensional position of the peripheral tissue surface on the basis of the distance information calculated from the pair of viewpoint images and the position and the orientation of the stereo camera detected by the viewpoint-position detecting portion. In addition, unlike an image acquired before the surgery, such as a CT image or the like, it is possible to acquire, in real time, the three-dimensional position of the peripheral tissue from the viewpoint images with which the interior of body cavity is observed during surgery.

The above-described first and second aspects may be provided with another medical device that can be inserted into the body cavity, wherein the organ measuring means may include: a specified-position detecting portion that detects a three-dimensional position of a distal end of the other medical device in the interior of the body cavity; and an instructing portion that is manipulated by the operator, wherein the non-interference-region setting portion may store the three-dimensional position detected by the specified-position detecting portion when the instructing portion is manipulated, and may set a non-interference region such that a surface including the stored three-dimensional position serves as an boundary surface of the non-interference region.

By doing so, the operator repeatedly manipulates the instructing portion while moving the other medical device in the interior of the body cavity, and thus, the operator can set a non-interference region in which positions of the operator own specification serve as a boundary surface thereof.

In the above-described first and second aspects, the organ measuring means may include a lightwave range finder that is provided at the distal end of the medical device so as to be rotatable about the axis in the longitudinal direction of the medical device and that scans measurement light in a one-dimensional direction that intersects the longitudinal direction.

By doing so, the distances to the individual positions of the peripheral tissue surface are measured by two-dimensionally scanning the measurement light over the peripheral tissue surface by radiating the measurement light onto the peripheral tissue while rotating the lightwave range finder, and thus, it is possible to measure the three-dimensional positions of the individual positions of the peripheral tissue surface. In addition, it is possible to accurately measure the three-dimensional position of the peripheral tissue even in locations with protrusions and depressions in the peripheral tissue or deeper locations in the interior of the body cavity.

In the above-described first and second aspects, the non-interference-region setting portion may assume a dimension of the organ to be a greater than a dimension determined on the basis of the three-dimensional position measured by the organ measuring means, and may set a non-interference region such that an outline of the organ having the assumed dimension serves as an boundary surface of the non-interference region.

In the above-described first and second aspects, the medical-device-occupying-region calculating portion may assume a dimension of the medical device to be a greater than the actual dimension of the medical device, and may set a medical-device occupying region such that an outline of the medical device having the assumed dimension serves as an boundary surface of the medical-device occupying region.

By doing so, because interference between the medical device and the peripheral tissue is predicted when the spacing between the medical device and the peripheral tissue becomes less than the predetermined value, it is possible to more reliably prevent interference between the medical device and the peripheral tissue.

The above-described first and second aspects may be provided with a plurality of the medical devices, wherein the medical-device detecting means may detect the three-dimensional position of each of the plurality of medical devices, the medical-device-occupying-region calculating portion may calculate the medical-device occupying region for each of the plurality of medical devices, and the interference predicting portion may predict interference among the plurality of medical devices on the basis of the positional relationships among the plurality of medical-device occupying regions calculated by using the medical-device occupying regions.

By doing so, it is possible to prevent not only interference between the medical device and the peripheral tissue but also interference between medical devices in the interior of the body cavity.

In the above-described first and second aspects, the medical device may be an endoscope or a treatment tool.

A third aspect of the present invention is a medical-device-interference avoidance method including: an organ measuring step of measuring a three-dimensional position of an organ in the interior of a body cavity of a living body; a non-interference-region setting step of setting, on the basis of the three-dimensional position of the organ measured in the organ measuring step, a non-interference region, which is a space in the interior of the body cavity excluding the organ; a medical-device detecting step of detecting a three-dimensional position of a medical device inserted into the interior of the body cavity; a medical-device-occupying-region calculating step of calculating, on the basis of the three-dimensional position of the medical device detected in the medical-device detecting step, a medical-device occupying region that is occupied by the medical device in the interior of the body cavity; an interference predicting step of predicting interference between the medical device and the organ on the basis of the positional relationship between the non-interference region set in the non-interference-region setting step and the medical-device occupying region calculated in the medical-device-occupying-region calculating step; and an alerting step of alerting an operator when interference between the medical device and the organ is predicted in the interference predicting step.

A fourth aspect of the present invention is a medical-device-interference avoidance method including: an organ measuring step of measuring a three-dimensional position of an organ in the interior of a body cavity of a living body; a non-interference-region setting step of setting, on the basis of the three-dimensional position of the organ measured in the organ measuring step, a non-interference region, which is a space in the interior of the body cavity excluding the organ; a medical-device detecting step of detecting a three-dimensional position of a medical device inserted into the interior of the body cavity; a medical-device-occupying-region calculating step of calculating, on the basis of the three-dimensional position of the medical device detected in the medical-device detecting step, a medical-device occupying region that is occupied by the medical device in the interior of the body cavity; an interference predicting step of predicting interference between the medical device and the organ on the basis of the positional relationship between the non-interference region set in the non-interference-region setting step and the medical-device occupying region calculated in the medical-device-occupying-region calculating step; and a medical-device restricting step of applying a restriction on the operation of the medical device when interference between the medical device and the organ is predicted in the interference predicting step.

### {Advantageous Effects of Invention}

The present invention affords an advantage in that it is possible to reliably prevent, in advance, the occurrence of interference between a medical device and peripheral tissues in the body cavity.

### {Brief Description of Drawings}

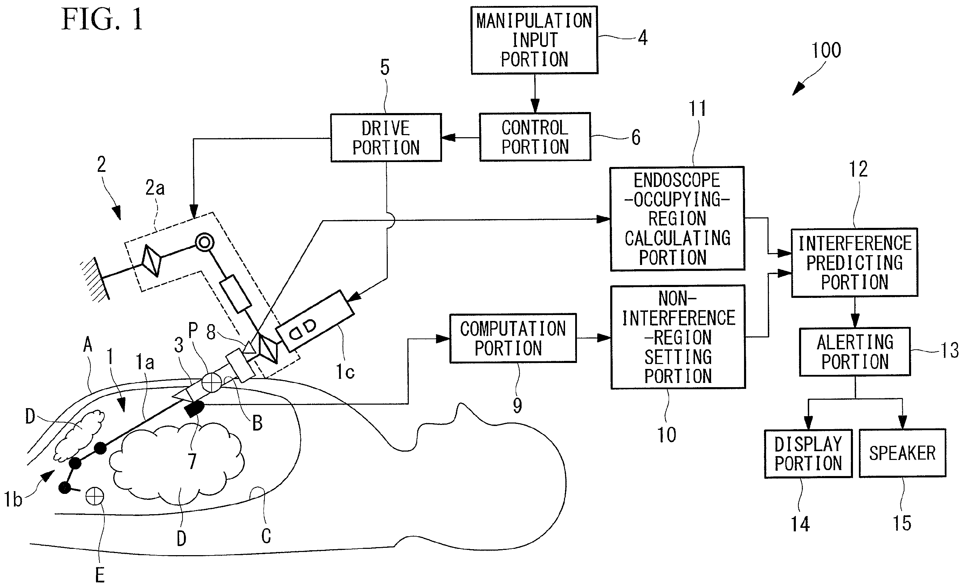

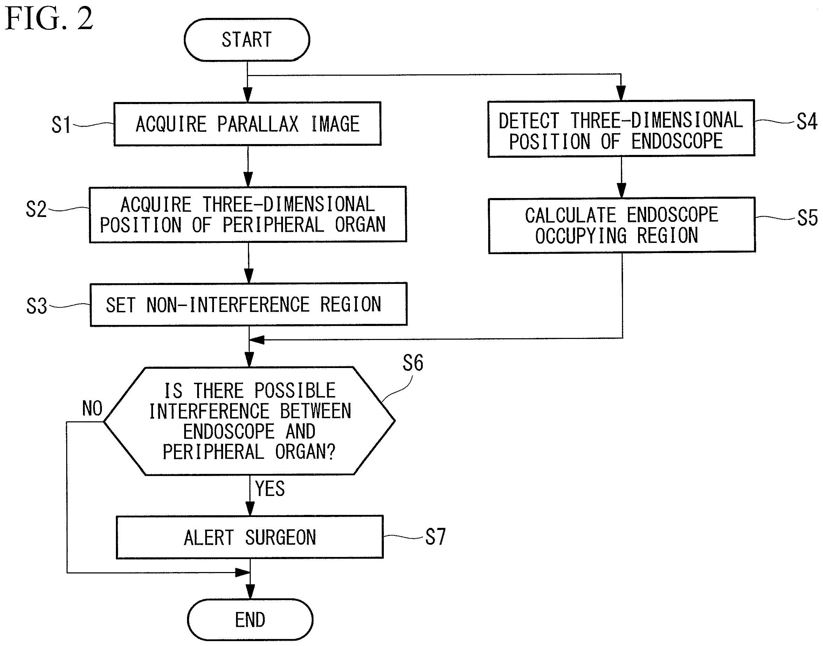

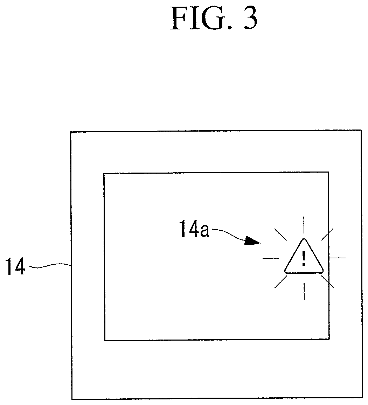

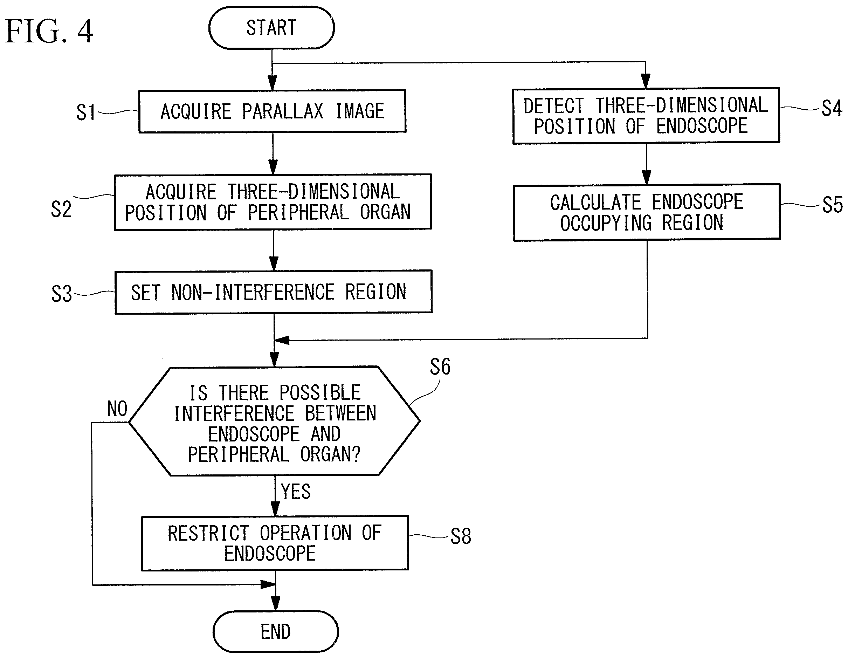

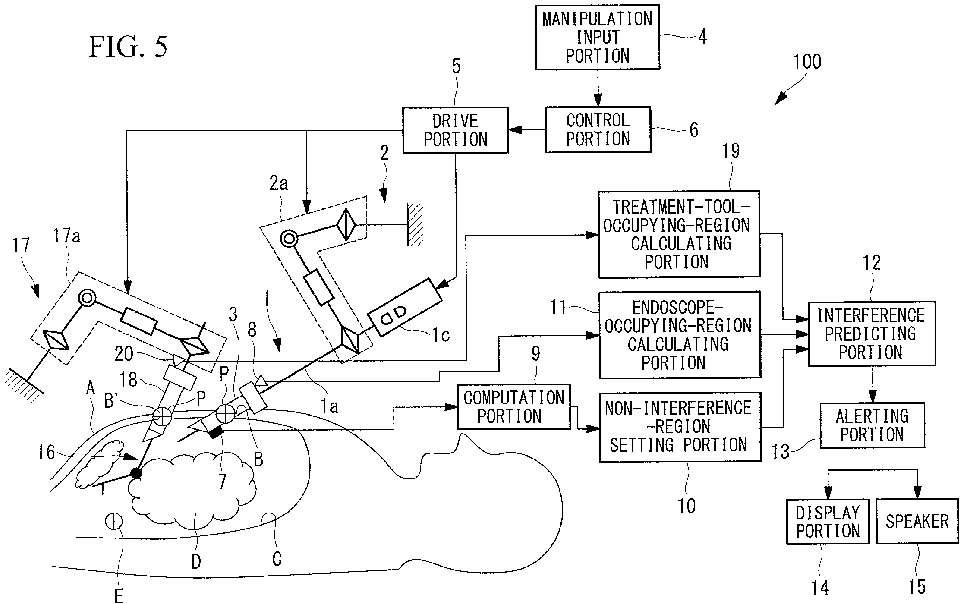

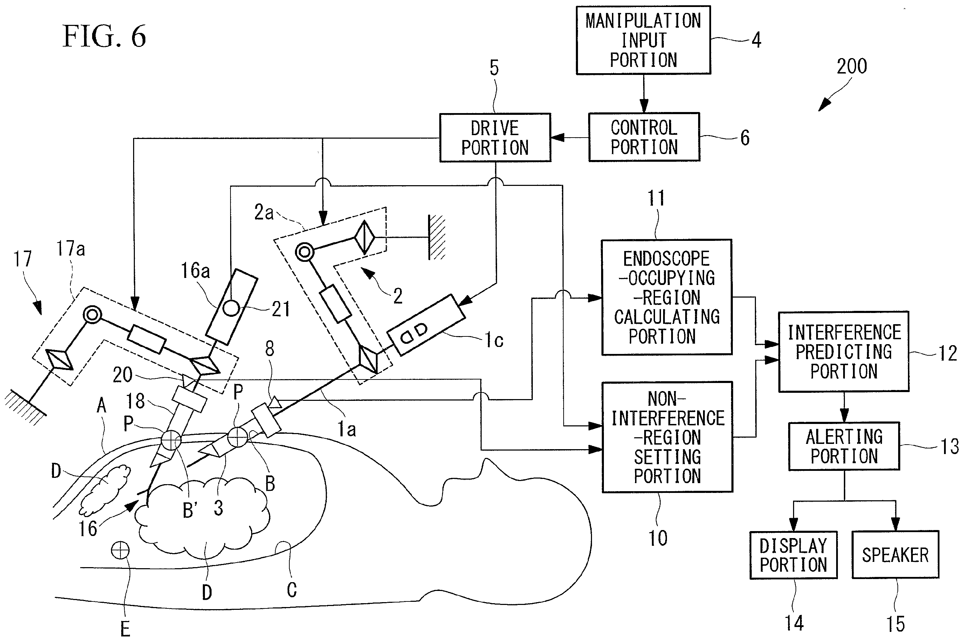

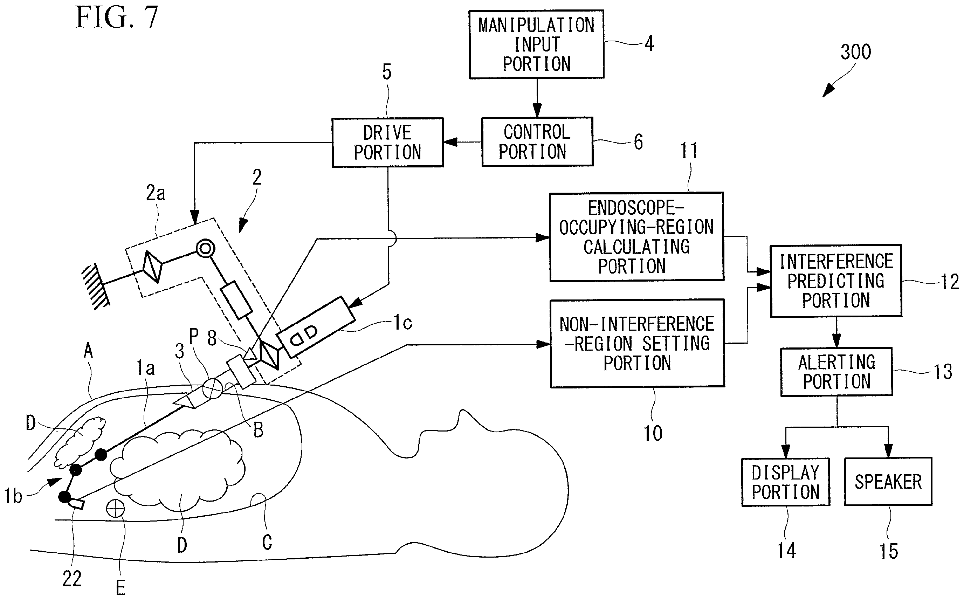

{Fig. 1} Fig. 1 is an overall configuration diagram of a surgical system according to a first embodiment of the present invention.
{Fig. 2} Fig. 2 is a flowchart for explaining an endoscope-interference avoidance method based on the surgical system of Fig. 1.
{Fig. 3} Fig. 3 is a diagram showing an example of a warning display displayed on a display portion in the surgical system of Fig. 1.
{Fig. 4} Fig. 4 is a flowchart for explaining a modification of the endoscope-interference avoidance method based on the surgical system of Fig. 1.
{Fig. 5} Fig. 5 is an overall configuration diagram showing a modification of the surgical system of Fig. 1.
{Fig. 6} Fig. 6 is an overall configuration diagram of a surgical system according to a second embodiment of the present invention.
{Fig. 7} Fig. 7 is an overall configuration diagram of a surgical system according to a third embodiment of the present invention.

### {Description of Embodiments}

### (First Embodiment)

A surgical system 100 according to a first embodiment of the present invention will be described below with reference to Figs. 1 to 5.

As shown in Fig. 1, the surgical system 100 according to this embodiment is provided with an endoscope (medical device) 1 that can be inserted into an abdominal cavity (body cavity) C via a hole B formed in a body wall A, an external arm 2 that holds a base-end portion of the endoscope 1 outside the body, a trocar 3 that supports the endoscope 1 in a state in which the endoscope 1 is inserted into the hole B, a manipulation input portion 4 manipulated by a surgeon (operator), a drive portion 5 that drives a bending portion 1b (described later) of the endoscope 1 and a joint portion 2a (described later) of the external arm 2, and a control portion 6 that controls the drive portion 5 on the basis of the manipulation input to the manipulation input portion 4.

The endoscope 1 is provided with a long, thin inserted portion 1a that is inserted into the abdominal cavity C, the bendable bending portion 1b provided at a distal-end portion of the inserted portion 1a, and a gripping portion 1c provided at a base end of the inserted portion 1a.

The external arm 2 is provided with the joint portion 2a formed of a plurality of joints, and the endoscope 1 held at the distal end thereof is movable by driving the joint portion 2a.

The manipulation input portion 4 is configured so as to allow inputs for manipulating the bending portion 1b and those for manipulating the joint portion 2a. When the surgeon inputs manipulations, the manipulation input portion 4 transmits signals corresponding to those manipulations to the control portion 6. By controlling the drive portion 5 on the basis of the signals received from the manipulation input portion 4, the control portion 6 causes the bending portion 1b and the joint portion 2a to execute the operations corresponding to the manipulations input via the manipulation input portion 4.

The trocar 3 is a cylindrical member that has a through-hole into which the inserted portion 1a can be inserted, and is inserted into the abdominal cavity C via the hole B. By inserting the inserted portion 1a into the interior of this trocar 3, it is possible to hold the inserted portion 1a in the state in which the inserted portion 1a is inserted into the interior of the abdominal cavity C. In addition, the trocar 3 can be pivoted by using the position supported by the body wall A as a fulcrum P, and, by changing the angle at which the trocar 3 is inserted into the interior of the abdominal cavity C, it is possible to move the inserted portion 1a in a direction that intersects the longitudinal direction thereof in the interior of the abdominal cavity C.

Furthermore, the surgical system 100 according to this embodiment is provided with: a stereo camera (organ measuring means) 7 and a sensor group (medical-device detecting means, organ measuring means, viewpoint-position detecting portion) 8 that are provided in the trocar 3; a computation portion (organ measuring means) 9 that acquires the three-dimensional position of a peripheral tissue D positioned in the area surrounding the inserted portion 1a by using a parallax image of the interior of the body cavity C acquired by the stereo camera 7; a non-interference-region setting portion 10 that sets a non-interference region on the basis of the three-dimensional position of the peripheral tissue D acquired by the computation portion 9; an endoscope-occupying-region calculating portion 11 that calculates, on the basis of the detected values from the sensor group 8, an endoscope occupying region occupied by the inserted portion 1a in the interior of the abdominal cavity C; interference predicting portion 12 that predicts interference between the inserted portion 1a and the peripheral tissue D on the basis of the non-interference region set by the non-interference-region setting portion 10 and the endoscope occupying region calculated by the endoscope-occupying-region calculating portion 11; and an alerting portion 13 that alerts the surgeon when the interference predicting portion 12 has predicted interference between the inserted portion 1a and the peripheral tissue D.

The stereo camera 7 is provided at a distal-end portion of the trocar 3 and acquires a pair of viewpoint images that constitute a parallax image by capturing the viewing field in front of the distal end of the trocar 3 from two different viewpoints. The parallax image is an image used for stereo viewing of an imaging subject and contains three-dimensional positional information of the imaging subject. The stereo camera 7 transmits the acquired parallax image to the computation portion 9.

The sensor group 8 at least includes an orientation sensor that detects the orientation of the trocar 3, an inserted-amount sensor that detects the amount by which the inserted portion 1a is inserted into the trocar 3, and an angle sensor that detects the amount by which the inserted portion 1a is rotated in the circumferential direction. With these three types of sensors, it is possible to detect the position and the orientation of the stereo camera 7 in the abdominal cavity C and the three-dimensional position of the inserted portion 1a in the interior of the abdominal cavity C. The individual sensors individually transmit the detected values to the computation portion 9 and the endoscope-occupying-region calculating portion 11.

By using the parallax image received from the stereo camera 7, the computation portion 9 acquires, by means of calculations, the distances from the stereo camera 7 to individual positions of the surface of the peripheral tissue D contained in the parallax image. Then, by converting the acquired distances to a three-dimensional coordinate system, in which the fulcrum P is the origin, by using the detected values received from the sensor group 8, the computation portion 9 obtains three-dimensional positional coordinates of the individual positions of the surface of the peripheral tissue D. The computation portion 9 transmits the obtained three-dimensional positional coordinates to the non-interference-region setting portion 10.

The non-interference-region setting portion 10 sets a region positioned farther from the fulcrum P as compared with a surface defined by the three-dimensional positional coordinates (that is, the surface of the peripheral tissue D) received from the computation portion 9 as an organ occupying region occupied by the peripheral tissue D in the abdominal cavity C. Next, the non-interference-region setting portion 10 sets a region expanded from the organ occupying region in the individual directions by a predetermined value as interference region, and, furthermore, sets, in the space in the abdominal cavity C, the space from which the interference region is excluded as a non-interference region. In other words, the non-interference region is a space in which the surface located the predetermined value away from the surface of the peripheral tissue D serves as the boundary surface and in which the peripheral tissue D does not exist.

The endoscope-occupying-region calculating portion 11 receives the detected values from the sensor group 8 and acquires the bending direction and the bending angle of the bending portion 1b from the control portion 6. Then, the endoscope-occupying-region calculating portion 11 calculates, on the basis of the information acquired from the sensor group 8 and the control portion 6 and the dimensions of the inserted portion 1a stored in advance, an endoscope occupying region in the above-described three-dimensional coordinate system, that is, a region occupied by the inserted portion 1a in the abdominal cavity C, and transmits the calculated endoscope occupying region to the interference predicting portion 12.

The interference predicting portion 12 compares the endoscope occupying region with the non-interference region, and, in the case in which at least a portion of the endoscope occupying region is positioned outside the boundary surface of the non-interference region, that is, in the case in which there is a portion in the inserted portion 1a in which the spacing with respect to the peripheral tissue D is less than a predetermined value, predicts a possible interference between the inserted portion 1a and the peripheral tissue D, thus transmitting a caution signal to the alerting portion 13.

When the caution signal is received from the interference predicting portion 12, the alerting portion 13 alerts the surgeon by outputting a notification signal. It suffices so long as the notification signal is noticeable by at least one of the vision, the auditory sense, and the sense of touch of the surgeon. For example, the alerting portion 13 may display a warning display on the display portion 14, may turn on a lamp, or may output a warning sound from a speaker 15 to issue a notification about the possible interference between the inserted portion 1a and the peripheral tissue D. Alternatively, the alerting portion 13 may cause the gripping portion 1c gripped by the surgeon to vibrate.

Next, the operation of the thus-configured surgical system 100 will be described.

In order to treat an affected area E in the abdominal cavity C by using the surgical system 100 according to this embodiment, the trocar 3 is inserted into the hole B, and the inserted portion 1a is inserted into the abdominal cavity C via the interior of the trocar 3. Subsequently, the surgeon can move the entire endoscope 1 by manipulating the joint portion 2a of the external arm 2 by using the manipulation input portion 4. In addition, the surgeon can move the distal end of the endoscope 1 by manipulating the bending portion 1b of the endoscope 1 by using the manipulation input portion 4.

Fig. 2 shows a flowchart for explaining an endoscope-interference avoidance method based on the surgical system 100. The following processing is executed while the inserted portion 1a and the bending portion 1b are being manipulated in the abdominal cavity C, as described above.

Specifically, a parallax image of the interior of the abdominal cavity C is acquired by the stereo camera 7 placed in the interior of the abdominal cavity C (organ measuring step S1), the three-dimensional position of the surface of the peripheral tissue D that exists in the interior of the abdominal cavity C is acquired from the parallax image (organ measuring step S2), and a region that is positioned closer to the near side than the peripheral tissue D is in the interior of the abdominal cavity C is set as the non-interference region (non-interference-region setting step S3). Concurrently with the above-described steps S1 to S3, the sensor group 8 detects the three-dimensional position of the inserted portion 1a in the interior of the abdominal cavity C (medical-device detecting step S4), and the endoscope occupying region is calculated on the basis of the detected three-dimensional position of the inserted portion 1a and the bending direction and the bending angle of the bending portion 1b (medical-device-occupying-region calculating step S5).

Then, whether or not the entire endoscope occupying region is positioned in the non-interference region is monitored by the interference predicting portion 12 (interference predicting step S6), and, when at least a portion of the endoscope occupying region falls outside the non-interference region ("YES" in step S6), the notification signal is output from the interference predicting portion 12 (alerting step S7). The notification signal prompts the surgeon to take an action to avoid interference between the inserted portion 1a and the peripheral tissue D, and thus, the surgeon can take an action to avoid, in advance, interference between the inserted portion 1a and the peripheral tissue D, for example, more cautiously manipulating the inserted portion 1a, moving the inserted portion 1a away from the peripheral tissue D, or the like.

As has been described, with this embodiment, the accurate three-dimensional position of the peripheral tissue D is measured by using a parallax image of the interior of the abdominal cavity C, and the non-interference region, in which the inserted portion 1a can be operated without interfering with the peripheral tissue D, is accurately set. Then, on the basis of the positional relationship between the position of the inserted portion 1a in the interior of the abdominal cavity C, which is obtained by means of calculation, and the non-interference region, interference between the inserted portion 1a and the peripheral tissue D is accurately predicted. By doing so, there is an advantage in that the surgeon can be prompted to take an appropriate avoidance action by notifying the surgeon about a highly likely occurrence of interference between the inserted portion 1a and the peripheral tissue D, before that interference occurs.

In addition, unlike the case in which an image is captured before the surgery, such as a CT image or the like, there is an advantage in that, by using the stereo camera 7, it is possible to acquire the current three-dimensional position of the peripheral tissue D. In addition, in the related art, it has been difficult to check for interference with the peripheral tissue D because it has been impossible to observe a portion of the inserted portion 1a near a body wall A by using an image; with this embodiment, however, there is an advantage in that, even when a portion of the inserted portion 1a is near the body wall A, it is possible to avoid interference with the peripheral tissue D by predicting the interference.

In this embodiment, interference region is set to be larger than the actual peripheral tissue D, and a region excluding the interference region is set as the non-interference region; however, alternatively or in addition thereto, the endoscope-occupying-region calculating portion 11 may use dimensions that are greater than the actual dimensions of the inserted portion 1a in calculating the endoscope occupying region. By doing so also, interference between the inserted portion 1a and the peripheral tissue D can reliably be predicted and avoided before the interference occurs.

In this embodiment, when interference between the inserted portion 1a and the peripheral tissue D is predicted, the direction in which the surgeon should move the inserted portion 1a in order to avoid interference may be notified to the surgeon by means of the notification signal. For example, in the case in which moving the inserted portion 1a farther to the right would cause interference, as shown in Fig. 3, a warning display 14a may be displayed on the right side of the display portion 14. By doing so, the surgeon can easily recognize the direction in which he/she should move the inserted portion 1a in order to avoid interference.

In this embodiment, although the surgeon is alerted when interference between the inserted portion 1a and the peripheral tissue D is predicted, alternatively or in addition thereto, as shown in Fig. 4, the control portion (medical-device control portion) 6 may apply, regardless of the inputs to the manipulation input portion 4, restrictions on the operation of the inserted portion 1a, such as prohibiting the operation of the inserted portion 1a at least in the direction in which the inserted portion 1a would be brought closer to the peripheral tissue D or the like (medical-device restricting step S8). By doing so, it is possible to more reliably prevent interference between the inserted portion 1a and the peripheral tissue D.

As shown in Fig. 5, in this embodiment, a treatment tool 16 that can be inserted into the abdominal cavity C via a hole B' formed at a position different from that of the hole B may be provided, and interference between the inserted portion 1a and the treatment tool 16 in the interior of the abdominal cavity C may also be predicted.

Specifically, the surgical system 100 of Fig. 5 is additionally provided with another external arm 17, another trocar 18, and a treatment-tool-occupying-region calculating portion 19.

The external arm 17 is configured in the same manner as the external arm 2 and has a joint portion 17a.

The trocar 18 is configured in the same manner as the trocar 3, and is provided with a sensor group (medical-device detecting means) 20 that at least includes an orientation sensor that detects the orientation of the trocar 18, an inserted-amount sensor that detects the amount by which the treatment tool 16 is inserted into the trocar 18, and an angle sensor that detects the amount by which the treatment tool 16 is rotated in the circumferential direction. With these three types of sensor, it is possible to detect the three-dimensional position of the treatment tool 16 in the interior of the abdominal cavity C.

By using the same method as that used by the endoscope-occupying-region calculating portion 11 to calculate the endoscope occupying region, the treatment-tool-occupying-region calculating portion 19 calculates, on the basis of the detected values received from the sensor group 20 and the dimensions of the treatment tool 16 stored in advance, a treatment-tool occupying region in the above-described three-dimensional coordinate system, that is a region occupied by the treatment tool 16 in the abdominal cavity C, and transmits the calculated treatment-tool occupying region to the interference predicting portion 12.

The interference predicting portion 12 compares the endoscope occupying region with the treatment-tool occupying region, and, for example, in the case in which the distance between these two occupying regions is less than a predetermined value, predicts interference between the inserted portion 1a and the treatment tool 16 and transmits the caution signal to the alerting portion 13.

By doing so, it is possible to predict and avoid, in advance, not only interference between the inserted portion 1a and the peripheral tissue D, but also interference between the inserted portion 1a and the treatment tool 16, which are used in the interior of the abdominal cavity at the same time, and thus, it is possible to smoothly manipulate the inserted portion 1a and the treatment tool 16. In the case in which a plurality of trocars 3 and 18 are provided, as in this modification, the stereo camera 7 may be provided in the trocar 3 or 18. In addition, by using an endoscope 1 capable of stereoscopic observation instead of the stereo camera 7, a parallax image acquired by the endoscope 1 may be used to measure the three-dimensional position of the peripheral tissue D.

### (Second Embodiment)

Next, a surgical system 200 according to a second embodiment of the present invention will be described with reference to Fig. 6.

In this embodiment, configurations that are different from those of the first embodiment will mainly be described, configurations that are the same as those of the first embodiment will be assigned the same reference signs, and descriptions thereof will be omitted.

The surgical system 200 according to this embodiment differs from that of the first embodiment mainly in terms of the configuration related to setting of the non-interference region.

As shown in Fig. 6, specifically, the surgical system 200 is provided with the treatment tool 16, another external arm 17 that holds the base-end portion of the treatment tool 16 at the body exterior, and another trocar 18 that supports the treatment tool 16 in a state in which the treatment tool 16 is inserted into the interior of the abdominal cavity C.

The treatment tool 16 is provided with, in the gripping portion 16a provided at the base end thereof, an instructing portion 21, like a button, that is manipulated by the surgeon. When manipulated by the surgeon, the instructing portion 21 transmits a signal indicating this fact to the non-interference-region setting portion 10.

The non-interference-region setting portion 10 receives the detected values from the sensor group (specified-position detecting portion) 20 provided in the trocar 18, and calculates, on the basis of the received detected values and the dimensions of the treatment tool 16 stored in advance, the three-dimensional position of the distal end of the treatment tool 16 in the interior of the abdominal cavity C. The non-interference-region setting portion 10 stores the three-dimensional positions of the distal end of the treatment tool 16 when the signals are received from the instructing portion 21. Then, the non-interference-region setting portion 10 sets a non-interference region such that the surface in which the accumulated three-dimensional positions are connected with each other serves as the boundary surface of the non-interference region.

Next, the operation of the thus-configured surgical system 200 will be described.

In order to treat the affected area E in the abdominal cavity C by using the surgical system 200 according to this embodiment, the trocar 3 is inserted into the hole B, and the inserted portion 1a is inserted into the abdominal cavity C via the interior of the trocar 3. In addition, the trocar 18 is inserted into the hole B', and the treatment tool 16 is inserted into the abdominal cavity C via the interior of the trocar 18. Subsequently, the surgeon can move the entire endoscope 1 or the entire treatment tool 16 by manipulating the joint portion 2a or 17a of the external arm 2 or 17 by using the manipulation input portion 4. In addition, the surgeon can move the distal end of the endoscope 1 by manipulating the bending portion 1b of the endoscope 1 by using the manipulation input portion 4.

Here, in this embodiment, the surgeon sets the non-interference region by performing the following procedures instead of the above-described steps S1 to S3. Specifically, the surgeon manipulates the treatment tool 16 and manipulates the instructing portion 21 in the state in which the distal end of the treatment tool 16 is placed in the vicinity of a surface of the peripheral tissue D (organ measuring step). This procedure is repeated while moving the distal end of the treatment tool 16 farther into the abdominal cavity C along the surface of the peripheral tissue D. By doing so, the non-interference-region setting portion 10 stores the three-dimensional positions arranged along the surface of the peripheral tissue D in the vicinity thereof, and thus, a non-interference region in which the boundary surface thereof is located in the vicinity of the surface of the peripheral tissue D is set (non-interference-region setting step). Because other procedures are the same as steps S4 to S7 described in the first embodiment, descriptions thereof will be omitted.

As has been described above, with this embodiment, by allowing the surgeon himself/herself to manually set the boundary of the non-interference region by using the treatment tool 16, it is possible to set the boundary surface of the non-interference region so as to accurately trace the shape of the peripheral tissue D, even in locations with protrusions and depressions or deeper locations in the interior of the abdominal cavity C, which is difficult to observe by using the stereo camera 7. Therefore, in the case in which treatment is performed by inserting the inserted portion 1a into a particularly narrow, bent portion while bending the bending portion 1b, there is an advantage in that it is possible to more reliably avoid interference between the inserted portion 1a and the peripheral tissue D.

### (Third Embodiment)

Next, a surgical system 300 according to a third embodiment of the present invention will be described with reference to Fig. 7.

In this embodiment, configurations that are different from those of the first embodiment will mainly be described, configurations that are the same as those of the first embodiment will be assigned the same reference signs, and descriptions thereof will be omitted.

The surgical system 300 according to this embodiment differs from that of the first embodiment mainly in terms of the configuration related to setting of the non-interference region.

Specifically, as shown in Fig. 7, the surgical system 300 is provided with, instead of the stereo camera 7, a lightwave range finder (organ measuring means) 22 that is provided at the distal end of the inserted portion 1a. The lightwave range finder 22 radiates measurement light, such as laser light, while scanning in a one-dimensional direction that intersects the longitudinal direction of the inserted portion 1a, receives reflected light coming from the peripheral tissue D, and calculates the distanced to the peripheral tissue D on the basis of the phase of the received reflected light and the receiving time of the reflected light. The lightwave range finder 22 transmits the calculated distance to the non-interference-region setting portion 10.

In addition, a rotating portion (not shown) that is rotatable about the axis in the longitudinal direction of the inserted portion 1a is provided at the distal-end portion of the inserted portion 1a. The lightwave range finder 22 is provided so as to rotate together with the rotating portion as a single unit and is configured so as to allow the measurement light to be two-dimensionally scanned by rotating the scanning direction of the measurement light from the lightwave range finder 22 by 360° about the axis in the longitudinal direction of the inserted portion 1a by means of rotation of the rotating portion.

The non-interference-region setting portion 10 receives the detected values of the sensor group 8, also acquires the bending direction and the bending angle of the bending portion 1b from the control portion 6, and receives the distance from the lightwave range finder 22. Then, the non-interference-region setting portion 10 calculates, on the basis of the received information and the dimensions of the inserted portion 1a stored in advance, the three-dimensional position of the distal end of the inserted portion 1a in the interior of the abdominal cavity C. Furthermore, the non-interference-region setting portion 10 calculates, on the basis of the three-dimensional position of the distal end of the inserted portion 1a and the distance measured by the lightwave range finder 22, the three-dimensional positions of the individual positions of the surface of the peripheral tissue D, and sets a non-interference region such that a surface defined by the obtained three-dimensional positions of the surface of the peripheral tissue D serves as the boundary surface of the non-interference region.

Next, the operation of the thus-configured surgical system 300 will be described.

In this embodiment, the non-interference region is set by means of the following procedures instead of the above-described steps S1 to S3. Specifically, the surgeon manipulates the inserted portion 1a, and irradiates the surface of the peripheral tissue D while the measurement light from the lightwave range finder 22 provided at the distal end of the inserted portion 1a is two-dimensionally scanned (organ measuring step). By doing so, distances from the distal end of the inserted portion 1a to the individual positions of the surface of the peripheral tissue D are measured, the three-dimensional positions of the individual positions of the surface of the peripheral tissue D are acquired on the basis of the measured distances, and the non-interference region is set on the basis of the obtained three-dimensional positions of the peripheral tissue D. The distance measurement by the lightwave range finder 22 and the non-interference-region setting are performed in association with the movement of the inserted portion 1a in the interior of the abdominal cavity C. Because other procedures are the same as steps S4 to S7 described in the first embodiment, descriptions thereof will be omitted.

As has been described above, with this embodiment, by measuring the distance from the distal end of the inserted portion 1a to the peripheral tissue D by using the lightwave range finder 22 and by setting the boundary of the non-interference region on the basis of the obtained distance, it is possible to set the boundary surface of the non-interference region so as to accurately trace the shape of the peripheral tissue D, even in locations with protrusions and depressions or deeper locations in the interior of the abdominal cavity C, which are difficult to observe by using the stereo camera 7. Therefore, in the case in which treatment is performed by inserting the inserted portion 1a into a particularly narrow, bent portion while bending the bending portion 1b, there is an advantage in that it is possible to more reliably avoid interference between the inserted portion 1a and the peripheral tissue D.

In this embodiment, by using an endoscope 1 capable of stereoscopic observation instead of the lightwave range finder 22, the distance from the distal end of the inserted portion 1a to the peripheral tissue D may be measured by using a parallax image acquired by using the endoscope 1.

In the first to third embodiments, although the endoscope 1 has mainly been described as the medical device, interference between the treatment tool and the peripheral tissue D may be predicted by using a treatment tool such as forceps or the like instead of the endoscope 1.

The invention of a medical-device-interference avoidance method according to the following Additional Items can be derived from the above-described first to third embodiments.

### (Additional Item 1)

A medical-device-interference avoidance method comprising:
an organ measuring step of measuring a three-dimensional position of an organ in the interior of a body cavity of a living body;
a non-interference-region setting step of setting, on the basis of the three-dimensional position of the organ measured in the organ measuring step, a non-interference region, which is a space in the interior of the body cavity excluding the organ;
a medical-device detecting step of detecting a three-dimensional position of a medical device inserted into the interior of the body cavity;
a medical-device-occupying-region calculating step of calculating, on the basis of the three-dimensional position of the medical device detected in the medical-device detecting step, a medical-device occupying region that is occupied by the medical device in the interior of the body cavity;
an interference predicting step of predicting interference between the medical device and the organ on the basis of the positional relationship between the non-interference region set in the non-interference-region setting step and the medical-device occupying region calculated in the medical-device-occupying-region calculating step; and
an alerting step of alerting an operator when interference between the medical device and the organ is predicted in the interference predicting step.

### (Additional Item 2)

A medical-device-interference avoidance method comprising:
an organ measuring step of measuring a three-dimensional position of an organ in the interior of a body cavity of a living body;
a non-interference-region setting step of setting, on the basis of the three-dimensional position of the organ measured in the organ measuring step, a non-interference region, which is a space in the interior of the body cavity excluding the organ;
a medical-device detecting step of detecting a three-dimensional position of a medical device inserted into the interior of the body cavity;
a medical-device-occupying-region calculating step of calculating, on the basis of the three-dimensional position of the medical device detected in the medical-device detecting step, a medical-device occupying region that is occupied by the medical device in the interior of the body cavity;
an interference predicting step of predicting interference between the medical device and the organ on the basis of the positional relationship between the non-interference region set in the non-interference-region setting step and the medical-device occupying region calculated in the medical-device-occupying-region calculating step; and
a medical-device restricting step of applying a restriction on the operation of the medical device when interference between the medical device and the organ is predicted in the interference predicting step.

### (Additional Item 3)

A medical-device-interference avoidance method according to Additional Item 2 further comprising:
an alerting step of alerting an operator when interference between the medical device and the organ is predicted in the interference predicting step.

### (Additional Item 4)

A medical-device-interference avoidance method according to Additional Item 1 or 3, wherein a notification signal that is noticeable by at least one of the vision, the auditory sense, and the sense of touch of the operator is output in the alerting step.

### (Additional Item 5)

A medical-device-interference avoidance method according to any one of Additional Items 1 to 4, wherein, in the organ measuring step, a pair of viewpoint images are acquired by capturing the interior of the body cavity from two different viewpoints and a three-dimensional position of the organ in the interior of the body cavity is calculated on the basis of the pair of acquired viewpoint images.

### (Additional Item 6)

A medical-device-interference avoidance method according to any one of Additional Items 1 to 4,
wherein, in the organ measuring step, a three-dimensional position of a distal end of another medical device in the interior of the body cavity is detected when instructed by the operator, and
in the non-interference-region setting step, the three-dimensional position detected in the organ measuring step is stored, and a non-interference region is set such that a surface including the stored three-dimensional position serves as an boundary surface of the non-interference region.

### (Additional Item 7)

A medical-device-interference avoidance method according to any one of Additional Items 1 to 4, wherein, in the organ measuring step, a lightwave range finder is used, which is provided at the distal end of the medical device so as to be rotatable about the axis in the longitudinal direction of the medical device and which scans measurement light in a one-dimensional direction that intersects the longitudinal direction.

### (Additional Item 8)

A medical-device-interference avoidance method according to any one of Additional Items 1 to 7, wherein, in the non-interference-region setting step, a dimension of the organ is assumed to be a greater than a dimension determined on the basis of the three-dimensional position measured in the organ measuring step, and a non-interference region is set such that an outline of the organ having the assumed dimension serves as an boundary surface of the non-interference region.

### (Additional Item 9)

A medical-device-interference avoidance method according to any one of Additional Items 1 to 7, wherein, in the medical-device-occupying-region calculating step, a dimension of the medical device is assumed to be a greater than the actual dimension of the medical device, and a medical-device occupying region is set such that an outline of the medical device having the assumed dimension serves as an boundary surface of the medical-device occupying region.

### (Additional Item 10)

A medical-device-interference avoidance method according to any one of Additional Items 1 to 9,
wherein individual three-dimensional positions of a plurality of medical devices are detected in the medical-device detecting step,
the medical-device occupying region is calculated for each of the plurality of medical devices in the medical-device-occupying-region calculating step, and
interference among the plurality of medical devices is additionally predicted in the interference predicting step on the basis of the positional relationship among the plurality of medical-device occupying regions calculated by using the medical-device occupying regions.

### (Additional Item 11)

A medical-device-interference avoidance method according to any one of Additional Items 1 to 10, wherein the medical device is an endoscope or a treatment tool.

### {Reference Signs List}

1 endoscope (medical device)
1a inserted portion
1b bending portion
1c gripping portion
2, 17 external arm
2a, 17a joint portion
3, 18 trocar
4 manipulation input portion
5 drive portion
6 control portion (medical-device control portion)
7 stereo camera (organ measuring means)
8 sensor group (medical-device detecting means, organ measuring means, viewpoint-position detecting portion)
9 computation portion (organ measuring means)
10 non-interference-region setting portion
11 endoscope-occupying-region calculating portion (medical-device-occupying-region calculating portion)
12 interference predicting portion
13 alerting portion
14 display portion
15 speaker
16 treatment tool (medical device)
19 treatment-tool-occupying-region calculating portion (medical-device-occupying-region calculating portion)
20 sensor group (medical-device detecting means, specified-position detecting portion)
21 instructing portion
22 lightwave range finder (organ measuring means) 100 surgical system
A body wall
B hole
C abdominal cavity (body cavity)
D peripheral tissue
E affected area
S1, S2 organ measuring step
S3 non-interference-region setting step
S4 medical-device detecting step
S5 medical-device-occupying-region calculating step
S6 interference predicting step
S7 alerting step
S8 medical-device restricting step

## Claims

1. A surgical system comprising:
a medical device that can be inserted into an interior of a body cavity of a living body;
an organ measuring means for measuring a three-dimensional position of an organ in the interior of the body cavity;
a non-interference-region setting portion that sets, on the basis of the three-dimensional position of the organ measured by the organ measuring means, a non-interference region, which is a space in the interior of the body cavity excluding the organ;
a medical-device detecting means for detecting a three-dimensional position of the medical device in the interior of the body cavity;
a medical-device-occupying-region calculating portion that calculates, on the basis of the three-dimensional position of the medical device detected by the medical-device detecting means, a medical-device occupying region that is occupied by the medical device in the interior of the body cavity;
an interference predicting portion that predicts interference between the medical device and the organ on the basis of the positional relationship between the non-interference region set by the non-interference-region setting portion and the medical-device occupying region calculated by the medical-device-occupying-region calculating portion; and
an alerting portion that alerts an operator when interference between the medical device and the organ is predicted by the interference predicting portion.

2. A surgical system comprising:
a medical device that can be inserted into an interior of a body cavity of a living body;
an organ measuring means for measuring a three-dimensional position of an organ in the interior of the body cavity;
a non-interference-region setting portion that sets, on the basis of the three-dimensional position of the organ measured by the organ measuring means, a non-interference region, which is a space in the interior of the body cavity excluding the organ;
a medical-device detecting means for detecting a three-dimensional position of the medical device in the interior of the body cavity;
a medical-device-occupying-region calculating portion that calculates, on the basis of the three-dimensional position of the medical device detected by the medical-device detecting means, a medical-device occupying region that is occupied by the medical device in the interior of the body cavity;
an interference predicting portion that predicts interference between the medical device and the organ on the basis of the positional relationship between the non-interference region set by the non-interference-region setting portion and the medical-device occupying region calculated by the medical-device-occupying-region calculating portion; and
a medical-device control portion that applies a restriction on the operation of the medical device when interference between the medical device and the organ is predicted by the interference predicting portion.

3. A surgical system according to Claim 2, further comprising:
an alerting portion that alerts an operator when interference between the medical device and the organ is predicted by the interference predicting portion.

4. A surgical system according to Claim 1 or 3, wherein the alerting portion outputs a notification signal that is noticeable by at least one of the vision, the auditory sense, and the sense of touch of the operator.

5. A surgical system according to any one of Claims 1 to 4, further comprising:
a cylindrical trocar that is inserted into the interior of the body cavity via a hole formed in a body surface of the living body and into which the medical device can be inserted,
wherein the organ measuring means includes:
a stereo camera that is provided in a distal-end portion of the trocar and that acquires a pair of viewpoint images by capturing a viewing field in front of the distal end of the trocar from two viewpoints that are different from each other;
a viewpoint-position detecting portion that detects a position and an orientation of the stereo camera in the interior of the body cavity; and
a computation portion that acquires, by means of calculation, a three-dimensional position of the organ in the interior of the body cavity on the basis of the pair of viewpoint images acquired by the stereo camera and the position and the orientation of the stereo camera detected by the viewpoint-position detecting portion.

6. A surgical system according to any one of Claims 1 to 4, further comprising:
another medical device that can be inserted into the body cavity,
wherein the organ measuring means includes:
a specified-position detecting portion that detects a three-dimensional position of a distal end of the other medical device in the interior of the body cavity; and
an instructing portion that is manipulated by the operator,
wherein the non-interference-region setting portion stores the three-dimensional position detected by the specified-position detecting portion when the instructing portion is manipulated, and sets a non-interference region such that a surface including the stored three-dimensional position serves as an boundary surface of the non-interference region.

7. A surgical system according to any one of Claims 1 to 4, wherein the organ measuring means includes a lightwave range finder that is provided at the distal end of the medical device so as to be rotatable about the axis in the longitudinal direction of the medical device and that scans measurement light in a one-dimensional direction that intersects the longitudinal direction.

8. A surgical system according to any one of Claims 1 to 7, wherein the non-interference-region setting portion assumes a dimension of the organ to be a greater than a dimension determined on the basis of the three-dimensional position measured by the organ measuring means, and sets a non-interference region such that an outline of the organ having the assumed dimension serves as an boundary surface of the non-interference region.

9. A surgical system according to any one of Claims 1 to 7, wherein the medical-device-occupying-region calculating portion assumes a dimension of the medical device to be a greater than the actual dimension of the medical device, and sets a medical-device occupying region such that an outline of the medical device having the assumed dimension serves as an boundary surface of the medical-device occupying region.

10. A surgical system according to any one of Claims 1 to 9, further comprising:
a plurality of the medical devices,
wherein the medical-device detecting means detects the three-dimensional position of each of the plurality of medical devices,
the medical-device-occupying-region calculating portion calculates the medical-device occupying region for each of the plurality of medical devices, and
the interference predicting portion predicts interference among the plurality of medical devices on the basis of the positional relationships among the plurality of medical-device occupying regions calculated by using the medical-device occupying regions.

11. A surgical system according to any one of Claims 1 to 10, wherein the medical device is an endoscope or a treatment tool.

12. A medical-device-interference avoidance method comprising:
an organ measuring step of measuring a three-dimensional position of an organ in the interior of a body cavity of a living body;
a non-interference-region setting step of setting, on the basis of the three-dimensional position of the organ measured in the organ measuring step, a non-interference region, which is a space in the interior of the body cavity excluding the organ;
a medical-device detecting step of detecting a three-dimensional position of a medical device inserted into the interior of the body cavity;
a medical-device-occupying-region calculating step of calculating, on the basis of the three-dimensional position of the medical device detected in the medical-device detecting step, a medical-device occupying region that is occupied by the medical device in the interior of the body cavity;
an interference predicting step of predicting interference between the medical device and the organ on the basis of the positional relationship between the non-interference region set in the non-interference-region setting step and the medical-device occupying region calculated in the medical-device-occupying-region calculating step; and
an alerting step of alerting an operator when interference between the medical device and the organ is predicted in the interference predicting step.

13. A medical-device-interference avoidance method comprising:
an organ measuring step of measuring a three-dimensional position of an organ in the interior of a body cavity of a living body;
a non-interference-region setting step of setting, on the basis of the three-dimensional position of the organ measured in the organ measuring step, a non-interference region, which is a space in the interior of the body cavity excluding the organ;
a medical-device detecting step of detecting a three-dimensional position of a medical device inserted into the interior of the body cavity;
a medical-device-occupying-region calculating step of calculating, on the basis of the three-dimensional position of the medical device detected in the medical-device detecting step, a medical-device occupying region that is occupied by the medical device in the interior of the body cavity;
an interference predicting step of predicting interference between the medical device and the organ on the basis of the positional relationship between the non-interference region set in the non-interference-region setting step and the medical-device occupying region calculated in the medical-device-occupying-region calculating step; and
a medical-device restricting step of applying a restriction on the operation of the medical device when interference between the medical device and the organ is predicted in the interference predicting step.
